# EUROPEAN PATENT SPECIFICATION

(11) **EP 3 746 106 B1**
(45) Date of publication and mention of the grant of the patent: **27.03.2024**
(21) Application number: 19747023.0
(22) Date of filing: 31.01.2019
(51) Int. Cl.: A61K 31/55, A61K 31/675, A61K 38/05, A61P 27/02, A61K 31/683

(54) **NEUTRAL ENDOPEPTIDASE (NEP) AND HUMAN SOLUBLE ENDOPEPTIDASE (HSEP) INHIBITORS FOR PROPHYLAXIS AND TREATMENT OF EYE DISEASES**
INHIBITOREN DER NEUTRALEN ENDOPEPTIDASE (NEP) UND DER HUMANEN LÖSLICHEN ENDOPEPTIDASE (HSEP) ZUR PROPHYLAXE UND BEHANDLUNG VON AUGENERKRANKUNGEN
INHIBITEURS D'ENDOPEPTIDASE NEUTRE (NEP) ET D'ENDOPEPTIDASE HUMAINE SOLUBLE (HSEP) DESTINÉS À LA PROPHYLAXIE ET AU TRAITEMENT DE MALADIES OCULAIRES

(30) Priority: 31.01.2018 PL 42445218
(43) Date of publication of application: 09.12.2020
(73) Proprietor: Turski, Christopher, 24-300 Opole Lubelskie (PL)
(72) Inventor: Turski, Christopher, 24-300 Opole Lubelskie (PL)
(74) Representative: Gosmann, Martin
(86) International application number: PCT/PL2019/000012
(87) International publication number: WO 2019/151882

(56) References cited:
- WO-A1-2005/030795
- US-A- 5 952 327
- US-A1- 2005 153 936
- US-A1- 2005 153 936
- GOOD TRAVIS J ET AL: "The role of endothelin in the pathophysiology of glaucoma", EXPERT OPINION ON THERAPEUTIC TARGETS, vol. 14, no. 6, 1 June 2010 (2010-06-01), pages 647-654, XP055837598, UK ISSN: 1472-8222, DOI: 10.1517/14728222.2010.487065 Retrieved from the Internet: URL:https://www.tandfonline.com/doi/pdf/10 .1517/14728222.2010.487065?needAccess=true >
- ROSENTHAL RITA ET AL: "Endothelin antagonism as an active principle for glaucoma therapy : Endothelin antagonism for glaucoma therapy", BRITISH JOURNAL OF PHARMACOLOGY, vol. 162, no. 4, 21 January 2011 (2011-01-21), pages 806-816, XP055837628, UK ISSN: 0007-1188, DOI: 10.1111/j.1476-5381.2010.01103.x
- GANESH PRASANNA ET AL: "Endothelin, astrocytes and glaucoma", EXPERIMENTAL EYE RESEARCH, ACADEMIC PRESS LTD, LONDON, vol. 93, no. 2, 7 September 2010 (2010-09-07), pages 170-177, XP028290731, ISSN: 0014-4835, DOI: 10.1016/J.EXER.2010.09.006 [retrieved on 2010-09-16]
- TABRIZCHI R: "SLV-306 SLOVAY", CURRENT OPINION IN INVESTIGATIONAL DRUGS, PHARMAPRESS, US, vol. 4, no. 3, 1 March 2003 (2003-03-01), pages 329-332, XP009029392, ISSN: 1472-4472
- MEAD BEN ET AL: "Evaluating retinal ganglion cell loss and dysfunction", EXPERIMENTAL EYE RESEARCH, ACADEMIC PRESS LTD, LONDON, vol. 151, 12 August 2016 (2016-08-12), pages 96-106, XP029749514, ISSN: 0014-4835, DOI: 10.1016/J.EXER.2016.08.006
- SMITH C A ET AL: "Assessing retinal ganglion cell damage", EYE, vol. 31, no. 2, 1 February 2017 (2017-02-01), pages 209-217, XP055837687, GB ISSN: 0950-222X, DOI: 10.1038/eye.2016.295 Retrieved from the Internet: URL:https://www.nature.com/articles/eye201 6295.pdf>
- TORRIGLIA ALICIA ET AL: "Mechanisms of cell death in neurodegenerative and retinal diseases : common pathway?", CURRENT OPINION IN NEUROLOGY, vol. 29, no. 1, 1 February 2016 (2016-02-01), pages 55-60, XP055837689, GB ISSN: 1350-7540, DOI: 10.1097/WCO.0000000000000272
- DÍAZ-CORÁNGUEZ MÓNICA ET AL: "The inner blood-retinal barrier: Cellular basis and development", VISION RESEARCH, ELSEVIER, AMSTERDAM, NL, vol. 139, 27 June 2017 (2017-06-27), pages 123-137, XP085290685, ISSN: 0042-6989, DOI: 10.1016/J.VISRES.2017.05.009

## Description

### FIELD OF THE INVENTION

The invention relates to dual inhibitors of endothelin converting enzyme-1 (ECE-1), and neutral endopeptidase (NEP) and/or human soluble endopeptidase (hSEP) alone, for use in the prophylaxis and/or treatment of eye diseases. Reference is made to the following scientific and patent literature in regard of endothelin and said dual inhibitors of ECE-1, and NEP and/or hSEP:
- US 2005/153936 A1 (IKONOMIOOU HRISSANTHI [NL] ET AL) 14 July 2005 (2005-07-14), corresponding to EP 1706121 A1;
- GOOO TRAVIS J ET AL: "The role of endothelin in the pathophysiology of glaucoma", EXPERT OPINION ON THERAPEUTIC TARGETS, vol. 14, no. 6, 1 June 2010 (2010-06-01), pages 647-654, XP055837598, UK ISSN: 1472-8222, 001: 10.1517/14728222.2010.487065;
- ROSENTHAL RITA ET AL: "Endothelin antagonism as an active principle for glaucoma therapy: Endothelin antagonism for glaucoma therapy", BRITISH JOURNAL OF PHARMACOLOGY, vol. 162, no. 4, 21 January 2011 (2011-01-21), pages 806-816, XP055837628, UK ISSN: 0007-1188, 001: 10.1111/j.1476-5381.201 0.011 03.x;
- GANESH PRASANNA ET AL: "Endothelin, astrocytes and glaucoma", EXPERIMENTAL EYE RESEARCH. ACADEMIC PRESS LTD. LONDON vol. 93, no. 2, 7 September 2010 (2010-09-07), pages 170-177, XP028290731. ISSN: 0014-4835. 001: 10.1 016/J.EXER.201 0.09.006;
- TABRIZCHI R: "SLV-306 SLOVAY". CURRENT OPINION IN INVESTIGATIONAL DRUGS. PHARMAPRESS. US. vol. 4. no. 3. 1 March 2003 (2003-03-01), pages 329-332, XP009029392, ISSN: 1472-4472;
- US 5 952 327 A (WALOECK HARALO [OE] ET AL) 14 September 1999 (1999-09-14 ), corresponding to EP 0916679 A1;
- MEAO BEN ET AL: "Evaluating retinal ganglion cell loss and dysfunction", EXPERIMENTAL EYE RESEARCH, ACAOEMIC PRESS LTO, LONOON, vol. 151 , 12 August 2016 (2016-08-12), pages 96-106, XP029749514, ISSN: 0014-4835. 001: 10.1 016/J.EXER.2016.08.006;
- SMITH C A ET AL: "Assessing retinal ganglion cell damage", EYE, vol. 31, no. 2, 1 February 2017 (2017-02-01), pages 209-217, XP055837687, GB ISSN: 0950-222X, 001: 10.1 038/eye.2016.295;
- TORRIGL1A AL1CIA ET AL: "Mechanisms of cell death in neurodegenerative and retinal diseases : common pathway?", CURRENT OPINION IN NEUROLOGY, vol. 29, no. 1, 1 February 2016 (2016-02-01), pages 55-60, XP055837689, GB ISSN: 1350-7540, 001: 10.1 097/WCO. 0000000000000272;
- OIAZ-CORANGUEZ MONICA ET AL: "The inner blood-retinal barrier: Cellular basis and development", VISION RESEARCH, ELSEVIER, AMSTEROAM, NL, vol. 139, 27 June 2017 (2017-06-27), pages 123-137, XP085290685, ISSN: 0042-6989, 001: 10.1 016/J.VISRES.2017.05.009;

### SCIENTIFIC BACKGROUND OF THE INVENTION

An increased intraocular pressure and reduced ocular blood flow are the major risk factors for glaucoma in humans. Glaucoma is the most common optic nerve head neuropathy and is associated morphologically with loss of retinal ganglion cells and clinically with visual field deterioration. Treatment of glaucoma is based on lowering of intraocular pressure and preventing the occurrence or progression of optic neuropathy. No therapy is currently available to mitigate ocular blood flow changes during glaucoma, or to limit the death of retinal ganglion cells by means of apoptosis in the course of glaucoma (B.C. Chauhan (2008) Can. J. Ophthalmol. 43, 356-360).

Endothelin is the human body's most potent vasoactive peptide known to date. As connoted by the term vasoactive peptide, this peptide participates in the regulation of intraocular blood pressure by means of vasoconstrictor activity. Upon release it causes a decrease in ocular blood flow followed by pathology in the retina and the optic nerve head.

Endothelin, apart from its vasoconstrictor activity, participates in the regulation of intraocular pressure by means of affecting trabecular outflow, which is the main route for fluid outflow from the eye. Endothelin increases contractility of the trabecular meshwork and therefore decreases fluid outflow from the eye elevating intraocular pressure followed by pathology in the retina and the optic nerve head.

Endothelin, apart from affecting intraocular blood pressure and regulating intraocular fluid pressure, influences apoptosis of retinal ganglion cells acting via ET_{B} receptors and induces proliferation of human optic nerve head astrocytes acting via both ET_{A} and ET_{B} receptors (G. Prasanna, R. Krishnamoorthy, A.F. Clark, R.J. Wordinger & T. Yorio (2002) Invest. Ophthalmol. Vis. Sci. 43, 2704-2713).

Endothelin is a peptide composed of 21 amino acids that is synthesized and released by the endothelium. Endothelin is produced by cleavage of a Trp-Val bond in the precursor peptide big endothelin (Big ET-1). Endothelin converting enzyme-1 (ECE-1), a membrane-bound metalloprotease, catalyses proteolytic activation of Big endothelin-1 to ET-1 and constitutes a regulatory site controlling production of the active peptide.

Neutral endopeptidase (NEP), a zink metallopeptidase, degrades atrial natriuretic peptide (ANP) and constitutes a regulatory site controlling concentration of the active peptide. In serum-deprived pheochromocytoma cells, ANP inhibits apoptosis by causing cGMP elevation. Spreading depression in the cortex is followed by long lasting elevation of the ANP expression and intracellular cGMP concentration. Natriuretic peptide receptor NPrA regulates intracellular cGMP concentration by stimulating particulate guanylyl cyclase (pGC), which activates cGMP-dependent protein kinase G pathway.

Since up-regulation of ET-1 receptors and down-regulation of ANP receptors has been reported to occur in cells subjected to metabolic or oxidative insults, it prompted us to explore the effects of dual ECE/NEP inhibition on rodent cells undergoing apoptosis similar to those reported in humans during ophthalmic diseases such as during e.g. glaucoma.

### INDUSTRIAL BACKGROUND OF THE INVENTION

The invention relates to a novel use of benzazepine, benzoxazepine, benzothiazepine-N-acetic acid and phosphono-substituted benzazepinone derivatives having neutral endopeptidase (NEP) and/or human soluble endopeptidase (hSEP) inhibitory activity. The compounds of the invention are useful for the preparation of pharmaceutical compositions for prophylaxis and treatment of ophthalmic diseases.

The disclosure, not part of the claimed invention relates to the use of compounds disclosed in the patent EP1706121 B1 (see also US 2005/153936 A1) for the manufacture of medicaments giving a beneficial effect. A beneficial effect is disclosed herein or apparent to a person skilled in the art from the specification and general knowledge in the art. The disclosure, not part of the claimed invention also relates to the use of compounds of the invention for the manufacture of medicaments for treating or preventing a disease or condition. More particularly, the disclosure, not part of the claimed invention relates to a new use for the treatment of a disease or condition disclosed herein or apparent to a person skilled in the art from the specification and general knowledge in the art. In embodiments of the disclosure, not part of the claimed invention specific compounds disclosed herein are used for the manufacture of a medication.

EP1706121 B1 (see also US 2005/153936 A1) refers to the use of certain compounds with a combined inhibitory activity on both neutral endopeptidase, and/or human soluble endopeptidase (hSEP), and endothelin converting enzyme (ECE) for the treatment and/or prophylaxis of neurodegenerative diseases such as traumatic brain injury, acute disseminated encephalomyelitis, epilepsy related brain damage, spinal cord injury, bacterial or viral meningitis and meningoencephalitis, prion diseases, poisonings with neurotoxic compounds, and radiation-induced brain damage, and for prophylaxis of ischemic stroke, with the proviso that said pharmaceutical compositions do not contain an aldosterone receptor antagonist.

WO 2004/082637, filed on March 18, 2004, and published on September 30, 2004, discloses a method for the prophylaxis or treatment of a very large number of pathological conditions, comprising administering an aldosterone receptor antagonist and an ECE inhibitor. Among the pathological conditions listed are glaucoma, hypertensive or diabetic retinopathy, and elevated intraocular pressure. However, no disclosure regarding the benefit of preferred compounds in these indications was made.

The object of the present invention was to identify specific metalloprotease inhibitors which are of therapeutic value in the prophylaxis and/or treatment of eye diseases.

The object of the present invention is solved by providing a compound of the general formula (1), 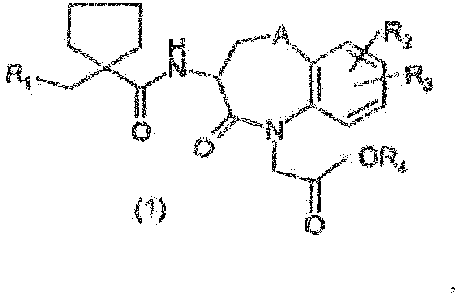 for use in the prophylaxis and/or treatment of optic and/or eye diseases selected from the group consisting of: (i) all forms of primary and secondary glaucoma; (ii) aquired macular disorders; (iii) optic neuropathy; (iv) optic neuritis; (v) uveitis; (vi) hereditary fundus dystrophies; (vii) retinal vascular diseases; (viii) scleritis and episcleritis; (ix) retinal detachments; (x) trauma to the eye globe; (xi) vitreous opacities; (xii) myopia and degenerative myopia; (xiii) postsurgical trauma; (xiv) dry eye disease; (xv) corneal disorders; and wherein in the compound of general formula (1)
R1 stands for a group with formula (2) or (3): 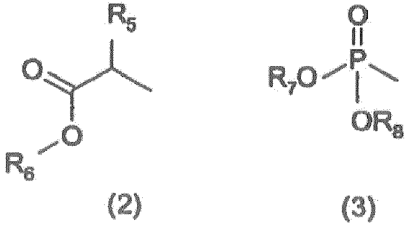
A represents CH2, O or S,
R2 and R3 independently represent hydrogen or halogen,
R4 and R6 independently represent hydrogen or a biolabile carboxylic ester forming group;
R5 is selected from the group consisting of (C1-C6)-alkoxy-(C1-C6)-alkyl which may be substituted by a (C1-C6)-alkoxy, phenyl-(C1-C6)-alkyl and phenyloxy-(C1-C6)-alkyl wherein the phenyl group may be substituted with (C1-C6)-alkyl, (C1-C6)-alkoxy or halogen, and naphtyl-(C1-C6)-alkyl,
R7 and R8 independently represent hydrogen or a group forming a biolabile phosphonic acid ester, and
all stereoisomers, as well as pharmaceutically acceptable salts thereof.
Surprisingly, it has been found that such benzazepine, benzoxazepine, benzothiazepine-N-acetic acid and phosphono-substituted benzazepinone derivatives of general formula (1) having neutral endopeptidase (NEP) and/or human soluble endopeptidase (hSEP) inhibitory activity prevent apoptosis in rodent models similar to that observed in the course of eye diseases in humans. This property makes compounds of general formula (1) useful for treatment and/or prophylaxis of optic and/or eye diseases selected from the group consisting of such diseases as (i) all forms of primary and secondary glaucoma, preferably primary open-angle glaucoma, normal-tension glaucoma, primary angle-closure glaucoma, pseudoexfoliation syndrome and glaucoma, pigment dispersion syndrome and glaucoma, neovascular glaucoma, inflammatory glaucoma, lens-related glaucoma, traumatic glaucoma, primary congenital glaucoma, iatrogenic induced glaucoma, and malignant glaucoma; (ii) aquired macular disorders, preferably age-related macular degeneration, idiopathic choroidal neovascularisation, central serous chorioretinopathy, vitreomacular interface disorders, idiopathic macular telangiectasia, cystoid macular oedema, and microcystic macular oedema; (iii) optic neuropathy, preferably anterior or posterior ischemic optic neuropathy; (iv) optic neuritis; (v) uveitis, preferably anterior uveitis, intermediate uveitis, posterior uveitis, and panuveitis; (vi) hereditary fundus dystrophies, preferably cone dystrophy, cone-rod dystrophy, rod dystrophy, Stargardt's disease, Bietti's crystalline comeoretinal dystrophy, familial benign fleck retina, Best vitelliform macular dystrophy, adult-onset vitelliform macular dystrophy, North Carolina macular dystrophy, familial dominant drusen, and concentric annular macular dystrophy; (vii) retinal vascular diseases, preferably diabetic retinopathy, non-diabetic retinopathy, retinal venous occlusive disease, retinal arterial occlusive disease, ocular ischaemic syndrome, hypertensive eye disease, sickle cell retinopathy, thalassemia retinopathy, retinopathy of prematurity, retinal artery macroaneurysm, primary retinal telangiectasia, Bales disease, and radiation retinopathy; (viii) scleritis and episcleritis; (ix) retinal detachments; (x) trauma to the eye globe; (xi) vitreous opacities, preferably vitreous hemorrhage, and asteroid hyalosis; (xii) myopia and degenerative myopia; (xiii) postsurgical trauma, preferably mechanical trauma due to conventional surgery, thermotrauma due to laser surgery, and trauma induced by cryosurgery; (xiv) dry eye disease; (xv) corneal disorders, preferably abrasions, lacerations, ulcerations, dystrophies, opacities, endothelial and epithelial decompensation, post-surgical oedema, corneal degenerations, corneal vascularisation; and corneal ectasias, preferably keratoconus.

The compounds of the invention are known from the European patents EP 0 733 642, EP 0 916 679 and EP 1 468 010, also containing detailed syntheses thereof.

To the invention belong all compounds for use having formula (1), racemates, mixtures of diastereomers and the individual stereoisomers, and also include pharmaceutically acceptable salts thereof. Thus compounds for use in which the substituents on potentially asymmetrical carbon atoms are in either the R-configuration or the S-configuration belong to the invention.

Pharmaceutically acceptable salts may be obtained using standard procedures well known in the art, for example by mixing a compound of the present invention with a suitable metal cation or an organic base, for instance an amine.

This can be achieved by preparing the metal salt of the compounds for use with the general formula (1) as mentioned above wherein the metal ion is a lithium ion or a bivalent metal ion. Preferred bivalent metal salts are calcium, magnesium and zinc salts. Most preferred is the calcium salt.

The invention relates to the compound of general formula (1), as defined above, for use in treatment and/or prophylaxis of optic and/or eye diseases selected from the group consisting of (i) all forms of primary and secondary glaucoma, preferably primary open-angle glaucoma, normal-tension glaucoma, primary angle-closure glaucoma, pseudoexfoliation syndrome and glaucoma, pigment dispersion syndrome and glaucoma, neovascular glaucoma, inflammatory glaucoma, lens-related glaucoma, traumatic glaucoma, primary congenital glaucoma, iatrogenic induced glaucoma, and malignant glaucoma; (ii) aquired macular disorders, preferably age-related macular degeneration, idiopathic choroidal neovascularisation, central serous chorioretinopathy, vitreomacular interface disorders, idiopathic macular telangiectasia, cystoid macular oedema, and microcystic macular oedema; (iii) optic neuropathy, preferably anterior or posterior ischemic optic neuropathy; (iv) optic neuritis; (v) uveitis, preferably anterior uveitis, intermediate uveitis, posterior uveitis, and panuveitis; (vi) hereditary fundus dystrophies, preferably cone dystrophy, cone-rod dystrophy, rod dystrophy, Stargardt's disease, Bietti's crystalline comeoretinal dystrophy, familial benign fleck retina, Best vitelliform macular dystrophy, adult-onset vitelliform macular dystrophy, North Carolina macular dystrophy, familial dominant drusen, and concentric annular macular dystrophy; (vii) retinal vascular diseases, preferably diabetic retinopathy, non-diabetic retinopathy, retinal venous occlusive disease, retinal arterial occlusive disease, ocular ischaemic syndrome, hypertensive eye disease, sickle cell retinopathy, thalassemia retinopathy, retinopathy of prematurity, retinal artery macroaneurysm, primary retinal telangiectasia, Bales disease, and radiation retinopathy; (viii) scleritis and episcleritis; (ix) retinal detachments; (x) trauma to the eye globe; (xi) vitreous opacities, preferably vitreous hemorrhage, and asteroid hyalosis; (xii) myopia and degenerative myopia; (xiii) postsurgical trauma, preferably mechanical trauma due to conventional surgery, thermotrauma due to laser surgery, and trauma induced by cryosurgery; (xiv) dry eye disease; (xv) corneal disorders, preferably abrasions, lacerations, ulcerations, dystrophies, opacities, endothelial and epithelial decompensation, post-surgical oedema, corneal degenerations, corneal vascularisation; and corneal ectasias, preferably keratoconus, with the proviso that said pharmaceutical compositions do not contain an aldosterone receptor antagonist.

Further embodiments of the invention are defined in the dependent claims.

The invention particularly relates to the compound for use, as defined above, having general formula (4): 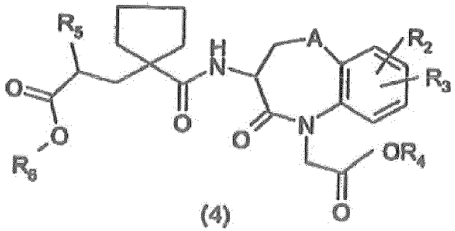 wherein the symbols have the meanings as given above for the compound of general formula (1).

More particular, the invention relates to the compound for use, as defined above, having general formula (5): 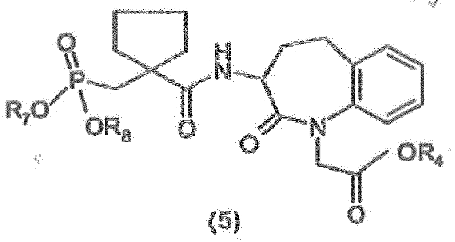 wherein the symbols have the meanings as given above for the compound of general formula (1).

The most preferred active substances for use according to the present invention are characterized in that the compound of general formula (1) for use are:
- (2R)-2-{[1-({[(3S)-1-(carboxymethyl)-2-oxo-2,3,4,5-tetrahydro-1*H*-1-benzazepin-3-yl]amino}carbonyl)cyclopentyl]methyl}-4-phenylbutanoic acid (6): 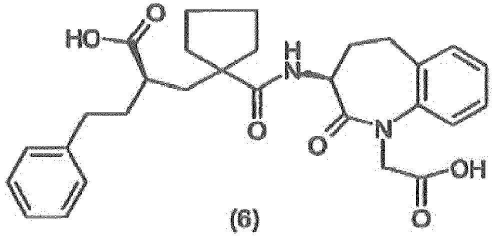
- (2R)-2-{[1-({[(3S)-1-(carboxymethyl)-2-oxo-2,3,4,5-tetrahydro-1*H*-1-benzazepin-3-yl]amino}carbonyl)cyclopentyl]methyl}-4-(1-naphthyl)butanoic acid (7): 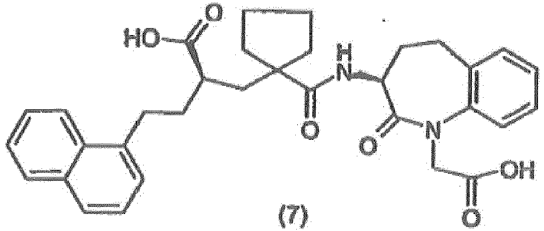
- tert-butyl-((3S)-3-{[(1-{[(benzyloxy)(ethoxy)phosphoryl]methyl)cyclopentyl) carbonyl]amino}-2-oxo-2,3,4,5-tetrahydro-1*H*-1-benzazepin-1-yl)acetate (8):

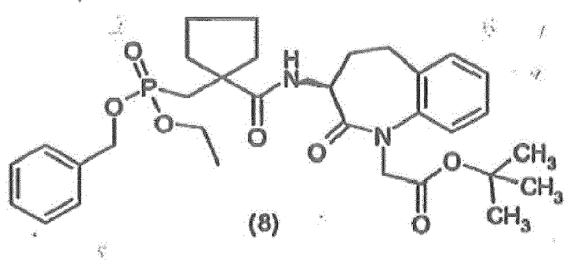

### PHARMACEUTICAL COMPOSITIONS

The compounds of general formula (1) for use according to the invention can be brought into forms suitable for administration by means of usual processes using auxiliary substances such as liquids or carrier materials. The pharmaceutical compositions of the invention may be administered either topically and/or systemically and in particular e.g. topically to the eye, periocularly in such manner as subconjunctival, subtenon, retrobulbar or peribulbar, intraocularly into the eye, but also enterally, orally, parenterally (intramuscularly or intravenously), subcutaneously or rectally. They can be administered in the form of solutions, suspensions, ointments (creams, gels, gel-forming solutions, sprays, ocular inserts/deposits, contact lenses), ocular implants but also tablets, capsules, softgels, powders, suppositories, nano-formulations or via iontophoresis, or by means of pharmaceutical compositions based on nanoparticle carrier systems. Suitable excipients for such formulations are the pharmaceutically customary liquid or solid fillers and extenders, solvents, emulsifiers, lubricants, flavorings, colorings and/or buffer substances. Frequently used auxiliary substances which may be mentioned are magnesium carbonate, titanium dioxide, lactose, mannitol and other sugars, talc, lactoprotein, gelatin, starch, cellulose and its derivatives, animal and vegetable oils such as fish liver oil, sunflower, groundnut or sesame oil, polyethylene glycol and solvents such as, for example, sterile water and mono- or polyhydric alcohols such as glycerol.

The compounds of general formula (1) for use according to the present invention are generally administered as pharmaceutical compositions. Types of pharmaceutical compositions that may be used include but are not limited to solutions, suspensions, ointments (creams, gels or sprays), but also tablets, chewable tablets, capsules, softgels, parenteral solutions, suppositories, and other types disclosed herein or apparent to a person skilled in the art from the specification and general knowledge in the art. These pharmaceutical compositions comprising the compound of general formula (1) for use according to the invention, in one aspect do not contain an aldosterone receptor antagonist. These pharmaceutical of said dual inhibitors of endothelin converting enzyme-1 (ECE-1), and neutral endopeptidase (NEP) and/or human soluble endopeptidase (hSEP) alone, with the proviso that said pharmaceutical compositions may optionally contain either one or more of carbonic anhydrase inhibitors such as e.g. brinzolamide, α₂-adrenergic agonists such as e.g. brimonidine, β-blockers such as e.g. timolol, prostaglandin analogs such as e.g. bimatoprost, rho kinase inhibitors such as e.g. netarsudil, adenosine A₁ receptor agonists such as e.g. trabodenoson, ET_{A} endothelin receptor antagonists such as e.g. sitaxentan, dual endothelin receptor antagonists such as e.g. bosentan, nitric oxide donors such as e.g. butanediol, parasympathomimetics such as e.g. pilocarpine, acetylcholine, catecholamines such as e.g. adrenaline, muscarinic receptor antagonists such as e.g. atropine, vascular endothelial growth factor inhibitors such as e.g. ranibizumab, corticosteroids such as e.g. dexamethasone, antibiotics such as e.g. vancomycin, tissue regenerating agents such as e.g. poly-carboxymethylglucose, vitamins and provitamins such as e.g. panthenol and retinyl palmitate, chemotherapeutic agents such as e.g. mitomycin, nonsteroidal anti-inflammatory drugs such as e.g. ketorolac, H₁ receptor antagonists such as e.g. cetirizine, monoclonal antibodies such as e.g. adalimumab, proteases such as e.g. ocriplasmin, immunosuppressive agents such as e.g. cyclosporine, or none.

In embodiments, comprising the compound of general formula (1) for use according tothe invention, a pharmaceutical pack or kit is provided comprising one or more containers filled with one or more of the ingredients of a pharmaceutical composition of the invention. Associated with such container(s) can be various written materials such as instructions for use, or a notice in the form prescribed by a governmental agency regulating the manufacture, use or sale of pharmaceuticals products, which notice reflects approval by the agency of manufacture, use, or sale for human or veterinary administration.

Very specific formulations suitable for the compounds of the invention have been described in the patent applications WO 03/068266 and WO 04/062692.

The following experimental models and examples are intended to further illustrate the invention in more detail, and therefore are not deemed to restrict the scope of the invention in any way.

### EYE DISEASES: DELAYED RETINAL CELL DEATH IN HUMANS AND EXPERIMENTAL MODELS

Apoptosis is a significant component of cell death in optic and/or eye diseases including (i) all forms of primary and secondary glaucoma, preferably primary open-angle glaucoma, normal-tension glaucoma, primary angle-closure glaucoma, pseudoexfoliation syndrome and glaucoma, pigment dispersion syndrome and glaucoma, neovascular glaucoma, inflammatory glaucoma, lens-related glaucoma, traumatic glaucoma, primary congenital glaucoma, iatrogenic induced glaucoma, and malignant glaucoma (R. Agarwal, S.K. Gupta, P. Agarwal, R. Saxena & S.S. Agrawal (2009) Indian J. Ophthalmol. 57, 257-266); (ii) aquired macular disorders, preferably age-related macular degeneration, idiopathic choroidal neovascularisation, central serous chorioretinopathy, vitreomacular interface disorders, idiopathic macular telangiectasia, cystoid macular oedema, and microcystic macular oedema (J.L. Dunaief, T. Dentchev, G.-S. Ying & A.H. Milam (2002) Arch. Ophthalmol. 120, 1435-1442); (iii) optic neuropathy, preferably anterior or posterior ischemic optic neuropathy (B.J. Slater, Z. Mehrabian, Y. Guo, A. Hunter & S.L. Bernstein (2008) Invest. Ophthalmol. Vis. Sci. 49, 3671-3676); (iv) optic neuritis (K.S. Shindler, E. Ventura, M. Dutt & A. Rostami (2008) Exp.Eye Res. 87, 208-213); (v) uveitis, preferably anterior uveitis, intermediate uveitis, posterior uveitis, and panuveitis (C.-C. Chan, D.M. Matteson, Q. Li, S.M. Whitcup & R.B. Nussenblatt (1997) Arch. Ophthalmol. 115, 1559-1567); (vi) hereditary fundus dystrophies, preferably cone dystrophy, cone-rod dystrophy, rod dystrophy, Stargardt's disease, Bietti's crystalline comeoretinal dystrophy, familial benign fleck retina, Best vitelliform macular dystrophy, adult-onset vitelliform macular dystrophy, North Carolina macular dystrophy, familial dominant drusen, and concentric annular macular dystrophy (H. Zhang, X. Li, X. Dai, J. Han, Y. Zhang, Y. Qi, Y. He, Y. Liu, B. Chang & J.J. Pang (2017) J. Ophthalmol. 2017, 1-13 9721362); (vii) retinal vascular diseases, preferably diabetic retinopathy, non-diabetic retinopathy, retinal venous occlusive disease, retinal arterial occlusive disease, ocular ischemic syndrome, hypertensive eye disease, sickle cell retinopathy, thalassemia retinopathy, retinopathy of prematurity, retinal artery macroaneurysm, primary retinal telangiectasia, Bales disease, and radiation retinopathy (G. Donati, A. Kapetanios, M. Dubois-Dauphin & C.J. Poumaras (2008) Acta Ophthalmol. 86, 302-306); (viii) scleritis or episcleritis (C. Heinz, N. Bograd, J. Koch & A. Heiligenhaus (2013) Graefes Arch. Clin. Exp. Ophthalmol. 251, 139-142); (ix) retinal detachments (J.G. Arroyo, L. Yang, D. Bula & D.F. Chen (2005) Am. J. Ophthalmol. 139, 605-610); (x) trauma to the eye globe (H.-C.H. Wang, J.-H. Choi, W.A. Greene, M.L. Plamper, H.E. Cortez, M. Chavko, Y. Li, J.J. Dalle Lucca & A.J. Johnson (2014) Military Med. 179, S34-S40); (xi) vitreous opacities vitreous haemorrhage and asteroid hyalosis (A. Alamri, H. Alkatan & I. Aljadaan (2016) Middle East African J. Ophthalmol. 23, 271-273); (xii) myopia and degenerative myopia (G.Z. Xu, W.W. Li & M.O. Tso (1996) Trans. Am. Ophthalmol. Soc. 94, 411-431); (xiii) postsurgical trauma, preferably mechanical trauma due to conventional surgery, thermotrauma due to laser surgery, and trauma induced by cryosurgery (A. Barak, T. Goldkom & L.S. Morse (2005) Invest. Ophthalmol. Vis. Sci. 46, 2587-2591; X. Liu, S. Ling, X. Gao, C. Xu & F.Wang (2013) JAMA Ophthalmol. 131, 1070-1072; D. Reichstein (2015) Curr. Opin. Ophthalmol. 26, 157-166); (xiv) dry eye disease (S. Yeh, X.J. Song, D.Q. Li, W. Farley, M.E. Stern & S.C. Pflugfelder (2003) Invest. Ophthalmol. Vis. Sci. 43, 124-129); (xv) corneal disorders, preferably such as abrasions, lacerations, ulcerations, dystrophies, opacities, endothelial and epithelial decompensation, post-surgical oedema, corneal degenerations, corneal vascularisation, and corneal ectasias, preferably such as keratoconus (R.M. Kaldawy, J. Wagner, S. Ching & G.M. Seigel (2002) Cornea 21, 206-209; N. Szentmary, B. Szende & I. Suveges (2005) Eur. J. Ophthalmol. 15, 17-22). In humans, apoptosis has been demonstrated to occur in the course of glaucoma in retinal ganglion cells by means of real-time imaging using annexin 5 labelled with fluorescent dye DY-776 (M.F. Cordeiro, E.M. Normando, M.J. Cardoso, S. Miodragovic, S. Jeylani, B.M. Davis, L. Guo, S. Ourselin, R. A'Hern & P.A. Bloom (2017) Brain 140, 1757-1767). To model this kind of apoptosis in a non-human experiment, rats are subjected to systemic administration of doxorubicin and apoptosis assessed in large organs such as the liver or heart (R. Gillet, G. Grimber, M. Bennoun, C. Caron de Fromentel, P. Briand & V. Joulin (2000) Oncogene 19, 3498-3507; L.L. Fan, G.P. Sun, W. Wei, Z.G. Wang, L. Ge, W.Z. Fu & H. Wang (2010) World J. Gastroenterol. 16, 1473-1481), or rats are subjected to the action of doxorubicin in the eye and apoptosis assessed in the retinal ganglion cells (I.M. Parhad, J.W. Griffin, A.W. Clark & J.F. Koves (1984) J. Neuropathol. Exp. Neurol. 43, 188-200). We used doxorubicin-induced apoptosis in the rat to assess whether systemic administration of compounds example prevents it.

### EXAMPLES

**Methods:** Wistar rats, 200-250 g, were anesthetized with chloral hydrate, 400 mg/kg, and osmotic minipumps, primed prior to implantation, were filled with either vehicle or example (6), representative for the compounds of the invention, in the dose of 60 mg/kg/d, and were implanted subcutaneously. Subsequently, rats (n=7) received doxorubicin in the dose of 5 mg/kg i.p. t.i.d. on days 1, 2 and 3, or rats (n=8) received doxorubicin in the dose of 5 µg or vehicle in the volume of 5 µl into the vitreous body of the eye by means of a Hamilton microsyringe over 5 min on the day 1. Rats were euthanized 5 days after the first i.p. injection of doxorubicin, or 5 days after the administration of doxorubicin into the vitreous body, and were transcardially perfused with a solution containing 4% paraformaldehyde in phosphate buffer. The livers and the eyes were subsequently removed and embedded in paraffin. To visualize apoptosis, a terminal deoxynucleotide transferase-mediated dUTP nick end-label (TUNEL) based staining was performed on 10 µm paraffin sections. The method of stereological disector (L.M. Cruz-Orive & E.R. Weibel (1990) Am. J. Physiol. 258, L148-L156) was used for quantification of apoptosis. An unbiased counting frame (0.05 x 0.05 mm, disector height 0.01 mm) was used for liver and (0.10 x 0.025 mm, disector height 0.01 mm) for the retinal ganglion cell layer for the sampling. The Nᵥ of TUNEL positive cells was determined with 8-10 dissectors and is expressed as number of TUNEL positive cells x 10²/mm³ for liver and as number of TUNEL positive cells x 10³/mm³ for retinal ganglion cells. Statistical evaluation was performed by means of Student's t-test (Table 1).

**Results:** Doxorubicin caused apoptosis either in the liver or in the retina as evidenced by the presence of TUNEL positive cells (Table 1). Example (6) conferred significant protection against doxorubicin induced apoptosis in the liver and in the retina as evidenced by reduction of Nᵥ of TUNEL positive cells vs vehicle-treated subjects (Table 1).

**Conclusions:** Compound (6) decreased density of apoptotic cells in the rat liver by 23.46 % and in the retina by 8.21 % (Table 1). The apoptosis induced by doxorubicin in the liver was characterized by many morphological features similar to those of apoptosis seen in the retinal ganglion cells.

**Table 1. Effect on doxorubicin-induced apoptosis.**

| | Doxorubicin +Vehicle Nᵥ | Doxorubicin +Example (6) Nᵥ |
|---|---|---|
| Liver | 10.36 ± 0.55 | 7.93 ± 0.25** |
| % | 100 | 76.54 |
| RGC | 36.65 ± 0.36 | 33.64 ± 0.96* |
| % | 100 | 91.79 |

| | | |
|---|---|---|
| *P<0.05, **P<0.01 vs doxorubicin + vehicle; Student's t-test; RGC, retinal ganglion cells; Nᵥ, number of TUNEL-positiv cells/mm³ ± SEM. | | |

## Claims

1. A compound of the general formula (1), 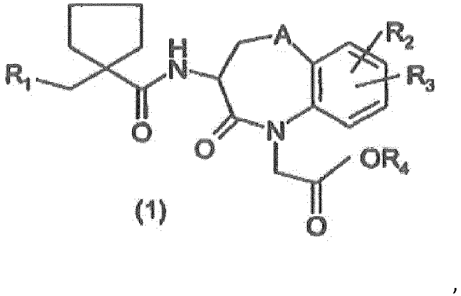
for use in the prophylaxis and/or treatment of optic and/or eye diseases selected from the group consisting of
(i) all forms of primary and secondary glaucoma;
(ii) aquired macular disorders;
(iii) optic neuropathy;
(iv) optic neuritis;
(v) uveitis;
(vi) hereditary fundus dystrophies;
(vii) retinal vascular diseases;
(viii) scleritis and episcleritis;
(ix) retinal detachments;
(x) trauma to the eye globe;
(xi) vitreous opacities;
(xii) myopia and degenerative myopia;
(xiii) postsurgical trauma;
(xiv) dry eye disease;
(xv) corneal disorders;
and wherein in the compound of general formula (1)
R1 stands for a group with formula (2) or (3): 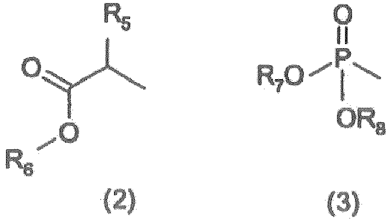
A represents CH2, O or S,
R2 and R3 independently represent hydrogen or halogen,
R4 and R6 independently repressent hydrogen or a biolabile carboxylic ester forming group;
R5 is selected from the group consisting of (C1-C6)alkoxy(C1-C6)alkyl which may be substituted by a (C1-C6) alkoxy, phenyl-(C1-C6)-alkyl and phenyloxy-(C1-C6)-alkyl wherein the phenyl group may be substituted with (C1-C6)alkyl, (C1-C6)-alkoxy or halogen, and naphtyl-(C1-C6)-alkyl,
R7 and R8 independently represent hydrogen or a group forming a biolabile phosphonic acid ester, and
all stereoisomers, as well as pharmaceutically acceptable salts thereof.

2. The compound for use according to claim 1, **characterized in that** the compound for use is a compound of the general formula (4), 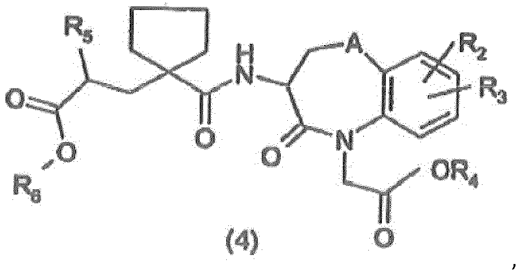 wherein the symbols A, and R₂ to R₆ have the meanings as given in claim 1, all stereoisomers, as well as pharmaceutically acceptable salts thereof.

3. The compound for use according to claim 1, **characterized in that** the compound for use is a compound of the general formula (5), 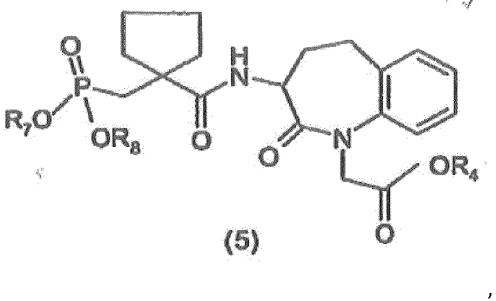 wherein the symbols R₄, and R₇ to R₈ have the meanings as given in claim 1, all stereoisomers, as well as pharmaceutically acceptable salts thereof.

4. The compound for use according to claim 1, **characterized in that** the compound for use is (2R)-2-{[1-({[(3S)-1-(carboxymethyl)-2-oxo-2,3,4,5-tetrahydro-1*H*-1-benzazepin-3-yl]amino}carbonyl)cyclopent-yl]methyl}-4-phenylbutanoic acid having formula (6): 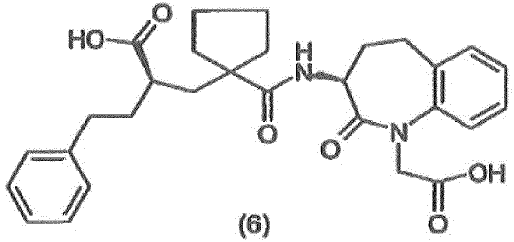 as well as pharmaceutically acceptable salts thereof.

5. The compound for use according to claim 1, **characterized in that** the compound for use is (2R)-2-{(1-({[(3S)-1-(carboxymethyl)-2-oxo-2,3,4,5-tetrahydro-1*H*-1-benzazepin-3-yl]amino}carbonyl)cyclopent-yl]methyl}-4-(1 - naphthyl)butanoic acid having formula (7): 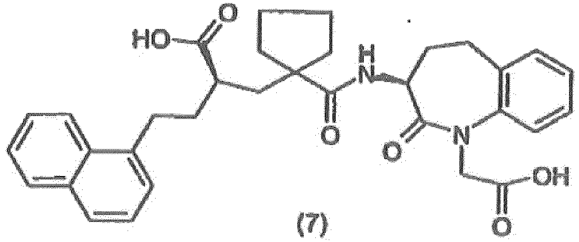 as well as pharmaceutically acceptable salts thereof.

6. The compound for use according to claim 1, **characterized in that** the compound for use is tert-butyl-((3S)-3-{[(1-{[(benzyloxy)(ethoxy)phosphoryl]-methyl}cyclopentyl)- carbonyl]amino)-2-oxo-2,3,4,5-tetrahydro-1*H*-1-benzazepin-1-yl)acetate having formula (8): 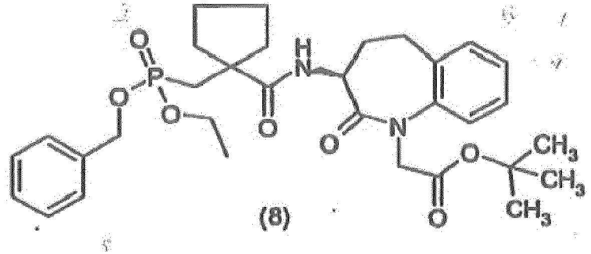 as well as pharmaceutically acceptable salts thereof.

7. The compound for use according to any of the claims 1-6, **characterized in that** the pharmaceutically acceptable salt is selected from the group consisting of the lithium salt, the calcium salt, the magnesium salt and the zinc salt, preferably in that the pharmaceutically acceptable salt is the calcium salt.

8. The compound for use according to any of the claims 1-7, **characterized in that** the compound is for use in the prophylaxis and/or treatment of (i) all forms of primary and secondary glaucoma, preferably primary open-angle glaucoma, normal-tension glaucoma, primary angle-closure glaucoma, pseudoexfoliation syndrome and glaucoma, pigment dispersion syndrome and glaucoma, neovascular glaucoma, inflammatory glaucoma, lens-related glaucoma, traumatic glaucoma, primary congenital glaucoma, iatrogenic induced glaucoma, or malignant glaucoma.

9. The compound for use according to any of the claims 1-7, **characterized in that** the compound is for use in the prophylaxis and/or treatment of (ii) aquired macular disorders, preferably age-related macular degeneration, idiopathic choroidal neovascularisation, central serous chorioretinopathy, vitreomacular interface disorders, idiopathic macular telangiectasia, cystoid macular oedema, or microcystic macular oedema.

10. The compound for use according to any of the claims 1-7, **characterized in that** the compound is for use in the prophylaxis and/or treatment of (iii) optic neuropathy, preferably anterior or posterior ischemic optic neuropathy; or of (iv) optic neuritis; or of (v) uveitis, preferably anterior uveitis, intermediate uveitis, posterior uveitis, or panuveitis; or of (viii) scleritis or episcleritis.

11. The compound for use according to any of the claims 1-7, **characterized in that** the compound is for use in the prophylaxis and/or treatment of (vi) hereditary fundus dystrophies, preferably cone dystrophy, cone-rod dystrophy, rod dystrophy, Stargardt's disease, Bietti's crystalline corneoretinal dystrophy, familial benign fleck retina, Best vitelliform macular dystrophy, adult-onset vitelliform macular dystrophy, North Carolina macular dystrophy, familial dominant drusen, or concentric annular macular dystrophy.

12. The compound for use according to any of the claims 1-7, **characterized in that** the compound is for use in the prophylaxis and/or treatment of (vii) retinal vascular diseases, preferably diabetic retinopathy, non-diabetic retinopathy, retinal venous occlusive disease, retinal arterial occlusive disease, ocular ischemic syndrome, hypertensive eye disease, sickle cell retinopathy, thalassemia retinopathy, retinopathy of prematurity, retinal artery macroaneurysm, primary retinal telangiectasia, Bales disease, or radiation retinopathy.

13. The compound for use according to any of the claims 1-7, **characterized in that** the compound is for use in the prophylaxis and/or treatment of (xii) myopia, or degenerative myopia; or of (xiv) dry eye disease.

14. The compound for use according to any of the claims 1-7, **characterized in that** the compound is for use in the treatment of (x) trauma to the eye globe; or of (xiii) postsurgical trauma, preferably mechanical trauma due to conventional surgery, thermotrauma due to laser surgery, or trauma induced by cryosurgery; or of (xi) vitreous opacities, preferably vitreous haemorrhage, or asteroid hyalosis; or of (ix) retinal detachments.

15. The compound for use according to any of the claims 1-7, **characterized in that** the compound is for use in the prophylaxis and/or treatment of (xv) corneal disorders, preferably abrasions, lacerations, ulcerations, dystrophies, opacities, endothelial and epithelial decompensation, post-surgical oedema, corneal degenerations, or corneal vascularisation; or of corneal ectasias, preferably keratoconus.

## Patentansprüche

1. Eine Verbindung der allgemeinen Formel (1), 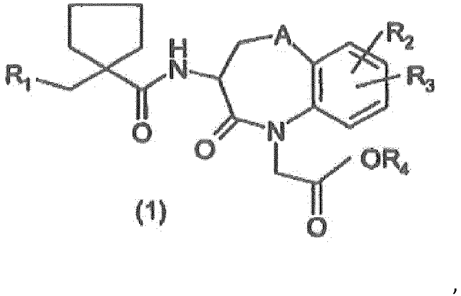
zur Verwendung in der Prophylaxe und/oder Behandlung von Seh- und/oder Augenerkrankungen ausgewählt aus der Gruppe bestehend aus:
(i) alle Formen des primären und sekundären Glaukoms;
(ii) erworbene Makulaerkrankungen;
(iii) Optikusneuropathie;
(iv) Optikusneuritis;
(v) Uveitis;
(vi) erbliche Fundusdystrophien;
(vii) Gefäßerkrankungen der Netzhaut;
(viii) Skleritis und Episkleritis;
(ix) Netzhautablösungen;
(x) Trauma des Augapfels;
(xi) Glaskörpertrübungen;
(xii) Myopie und degenerative Myopie;
(xiii) postoperatives Trauma;
(xiv) Erkrankung des trockenen Auges;
(xv) Hornhauterkrankungen;
und wobei in der Verbindung der allgemeinen Formel (1)
R1 für eine Gruppe mit der Formel (2) oder (3) steht: 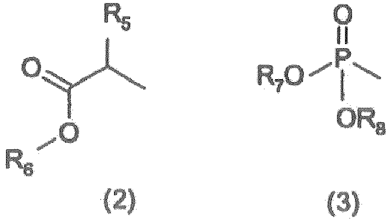
A CH₂, O oder S darstellt,
R2 und R3 unabhängig Wasserstoff oder Halogen darstellen,
R4 und R6 unabhängig Wasserstoff oder eine biolabile Carboxylesterbildende Gruppe darstellen;
R5 ausgewählt ist aus der Gruppe bestehend aus (C1-C6)-Alkoxy(C1-C6)-Alkyl welches substituiert sein kann durch ein (C1-C6)-Alkoxy, Phenyl-(C1-C6)-Alkyl und Phenyloxy-(C1-C6)-Alkyl, wobei die Phenylgruppe substituiert sein kann mit (C1-C6)-Alkyl, (C1-C6)-Alkoxy oder Halogen, und Naphtyl-(C1-C6)-Alkyl,
R7 und R8 unabhängig Wasserstoff oder eine biolabile Phosphonsäureester-bildende Gruppe darstellen, und
alle Stereoisomere, sowie pharmaceutisch akzeptable Salze davon.

2. Die Verbindung zur Verwendung nach Anspruch 1, **dadurch gekennzeichnet, dass** die Verbindung zur Verwendung eine Verbindung der allgemeinen Formel (4) ist, 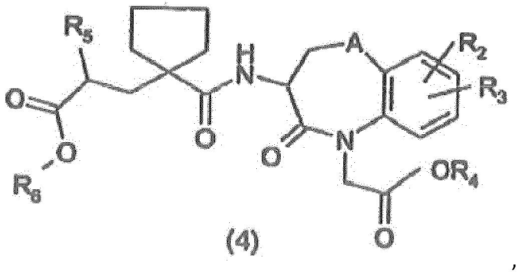 wobei die Symbole A, und R₂ bis R₅ die in Anspruch 1 angegebene Bedeutung haben, alle Stereoisomere, sowie pharmaceutisch akzeptable Salze davon.

3. Die Verbindung zur Verwendung nach Anspruch 1, **dadurch gekennzeichnet, dass** die Verbindung zur Verwendung eine Verbindung der allgemeinen Formel (5) ist, 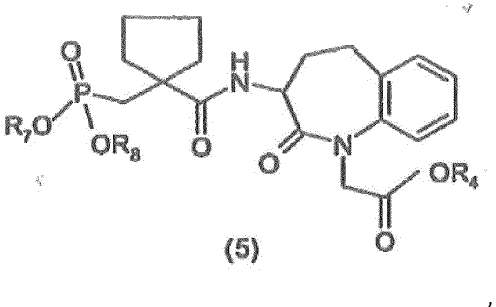 wobei die Symbole R₄, und R₇ to R₈ die in Anspruch 1 angegebene Bedeutung haben, alle Stereoisomere, sowie pharmaceutisch akzeptable Salze davon.

4. Die Verbindung zur Verwendung nach Anspruch 1, **dadurch gekennzeichnet, dass** die Verbindung zur Verwendung (2R)-2-{[1-({[(3S)-1-(Carboxymethyl)-2-oxo-2,3,4,5-tetrahydro-1*H*-1-benzazepin-3 - yl]amino}carbonyl)cyclopent-yl]methyl}-4-phenylbutansäure mit der Formel (6) ist: 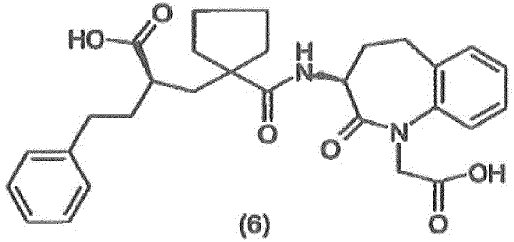 sowie pharmaceutisch akzeptable Salze davon.

5. Die Verbindung zur Verwendung nach Anspruch 1, **dadurch gekennzeichnet, dass** die Verbindung zur Verwendung (2R)-2-{(1-({[(3S)-1-(Carboxymethyl)-2-oxo-2,3,4,5-tetrahydro-1*H*-1-benzazepin-3-yl]amino}carbonyl)cyclopent-yl]methyl}-4-(1-naphthyl)butansäure mit der Formel (7) ist: 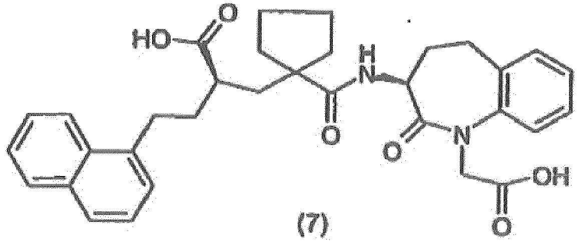 sowie pharmaceutisch akzeptable Salze davon.

6. Die Verbindung zur Verwendung nach Anspruch 1, **dadurch gekennzeichnet, dass** die Verbindung zur Verwendung tert-Butyl-((3S)-3-{[(1-{[(benzyloxy)(ethoxy)phosphoryl]methyl}cyclopentyl)-carbonyl]amino)-2-oxo-2,3,4,5-tetrahydro-1*H*-1-benzazepin-1-yl)acetat mit der Formel (8) ist: 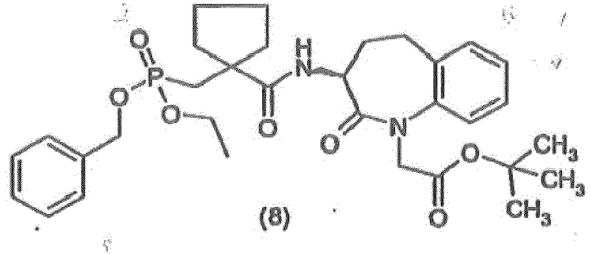 sowie pharmaceutisch akzeptable Salze davon.

7. Die Verbindung zur Verwendung nach einem der Ansprüche 1-6, **dadurch gekennzeichnet, dass** das pharmaceuticch akzeptable Salz ausgewählt aus der Gruppe bestehend aus dem Lithiumsalz, dem Kalziumsalz, dem Magnesiumsalz und dem Zinksalz, vorzugsweise dadurch, dass das pharmazeutisch akzeptable Salz das Kalziumsalz ist.

8. Die Verbindung zur Verwendung nach einem der Ansprüche 1-7, **dadurch gekennzeichnet, dass** die Verbindung bestimmt ist zur Verwendung in der Prophylaxe und/oder Behandlung von (i) allen Formen des primären und sekundären Glaukoms, vorzugsweise primäres Offenwinkelglaukom, Normaldruckglaukom, primäres Engwinkelglaukom, Pseudoexfoliationssyndrom und Glaukom, Pigmentdispersionssyndrom und Glaukom, neovaskuläres Glaukom, entzündliches Glaukom, linsenbedingtes Glaukom, traumatisches Glaukom, primäres angeborenes Glaukom, iatrogen induziertes Glaukom, oder malignes Glaukom.

9. Die Verbindung zur Verwendung nach einem der Ansprüche 1-7, **dadurch gekennzeichnet, dass** die Verbindung bestimmt ist zur Verwendung in der Prophylaxe und/oder Behandlung von (ii) erworbenen Makulaerkrankungen, vorzugsweise altersbedingte Makuladegeneration, idiopathische choroidale Neovaskularisation, zentrale seröse Chorioretinopathie, vitreomakuläre Grenzflächenstörungen, idiopathische makuläre Teleangiektasie, zystoides Makulaödem oder mikrozystisches Makulaödem.

10. Die Verbindung zur Verwendung nach einem der Ansprüche 1-7, **dadurch gekennzeichnet, dass** die Verbindung bestimmt ist zur Verwendung in der Prophylaxe und/oder Behandlung von (iii) Optikusneuropathie, vorzugsweise vordere oder hintere ischämische Optikusneuropathie; oder von (iv) Optikusneuritis; oder von (v) Uveitis, vorzugsweise anteriorer Uveitis, intermediärer Uveitis, posteriorer Uveitis oder Panuveitis; oder von (viii) Skleritis oder Episkleritis.

11. Die Verbindung zur Verwendung nach einem der Ansprüche 1-7, **dadurch gekennzeichnet, dass** die Verbindung bestimmt ist zur Verwendung in der Prophylaxe und/oder Behandlung von (vi) erblichen Fundusdystrophien, vorzugsweise Zapfendystrophie, Zapfen-Stäbchen-Dystrophie, Stäbchendystrophie, Morbus Stargardt, Bietti'sche kristalline korneoretinale Dystrophie, familiäre gutartige Retinaflecken, Best'sche vitelliforme Makuladystrophie, adulte vitelliforme Makuladystrophie, North Carolina Makuladystrophie, familiäre dominante Drusen, oder konzentrische ringförmige Makuladystrophie.

12. Die Verbindung zur Verwendung nach einem der Ansprüche 1-7, **dadurch gekennzeichnet, dass** die Verbindung bestimmt ist zur Verwendung in der Prophylaxe und/oder Behandlung von (vii) Gefäßerkrankungen der Netzhaut, vorzugsweise diabetische Retinopathie, nicht-diabetische Retinopathie, retinale venöse Verschlusskrankheit, retinale arterielle Verschlusskrankheit, okuläres Ischämiesyndrom, hypertensive Augenerkrankungen, Sichelzellretinopathie, Thalassämie-Retinopathie, Frühgeborenenretinopathie, Makroaneurysma der Netzhautarterie, primäre retinale Teleangiektasie, Eales-Erkrankung, oder Strahlenretinopathie.

13. Die Verbindung zur Verwendung nach einem der Ansprüche 1-7, **dadurch gekennzeichnet, dass** die Verbindung bestimmt ist zur Verwendung in der Prophylaxe und/oder Behandlung von (xii) Myopie und degenerative Myopie; oder von (xiv) Erkrankung des trockenen Auges.

14. Die Verbindung zur Verwendung nach einem der Ansprüche 1-7, **dadurch gekennzeichnet, dass** die Verbindung bestimmt ist zur Verwendung in der Prophylaxe und/oder Behandlung von (x) Trauma des Augapfels; oder von (xiii) postoperativem Trauma, vorzugsweise mechanisches Trauma aufgrund konventioneller Chirurgie, Thermotrauma aufgrund von Laserchirurgie oder Trauma durch Kryochirurgie; oder von (xi) Glaskörpertrübungen, vorzugsweise Glaskörperblutung oder Asteroide Hyalose; oder von (ix) Netzhautablösungen.

15. Die Verbindung zur Verwendung nach einem der Ansprüche 1-7, **dadurch gekennzeichnet, dass** die Verbindung bestimmt ist zur Verwendung in der Prophylaxe und/oder Behandlung von (xv) Hornhauterkrankungen, vorzugsweise Abschürfungen, Schnittwunden, Geschwüren, Dystrophien, Trübungen, endotheliale und epitheliale Dekompensation, postoperative Ödeme, Hornhautdegenerationen oder Hornhautvaskularisation; oder von Hornhautektasien, vorzugsweise Keratokonus.

## Revendications

1. Un composé de formule générale (1), 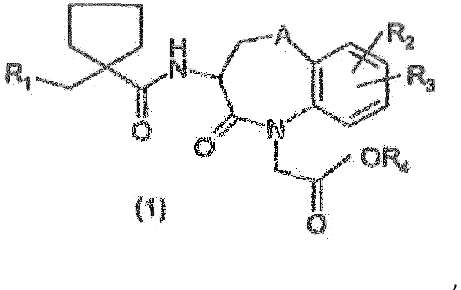
pour utilisation dans la prophylaxie et/ou le traitement de la vision optique et/ou des maladies oculaires, des maladies des yeux sélectionnées dans le groupe constitué de:
(i) toutes les formes de glaucome primaire et secondaire;
(ii) maladies maculaires acquises;
(iii) neuropathie optique;
(iv) névrite optique;
(v) uvéite;
(vi) dystrophies héréditaires du fond d'oeil,
(vii) maladies vasculaires de la rétine;
(viii) sclérite et épisclérite;
(ix) décollements de rétine;
(x) traumatisme du globe oculaire;
(xi) opacités vitréennes;
(xii) myopie et myopie dégénérative;
(xiii) traumatisme postopératoire;
(xiv) maladie de l'oeil sec;
(xv) maladies cornéennes;
et où dans le composé de formule générale (1)
R1 représente un groupe de formule (2) ou (3): 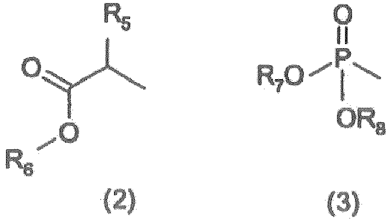
A représente CH₂, O ou S,
R2 et R3 représentent indépendamment l'hydrogène ou l'halogène,
R4 et R6 représentent indépendamment l'hydrogène ou un groupe formateur d'ester carboxyle biolabile;
R5 est choisi dans le groupe constitué de (C1-C6)-alcoxy-(C1-C6)-alkyle qui peut être substitué par un (C1-C6)-alcoxy, phényl-(C1-C6)-alkyle et phényloxy-(C1-C6)-alkyle, où le groupe phényle peut être substitué par un alkyle (C1-C6), un alcoxy (C1-C6) ou un halogène, et un naphtyl-alkyle (C1-C6);
R7 et R8 représentent indépendamment l'hydrogène ou un groupe formateur d'ester d'acide phosphonique biolabile, et
tous les stéréoisomères, ainsi que leurs sels pharmaceutiquement acceptables.

2. Le composé à utilisation selon la revendication 1, **caractérisé en ce que** le composé à utilisation est un composé représenté par la formule générale (4), 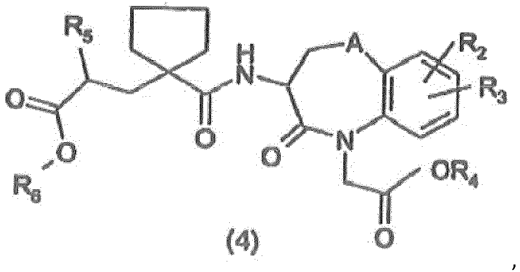 dans laquelle les symboles A et R2 à R6 ont la signification donnée dans la revendication 1, tous leurs stéréoisomères et sels pharmaceutiquement acceptables.

3. Le composé à utilisation selon la revendication 1, **caractérisé en ce que** le composé à utilisation est un composé représenté par la formule générale (5), 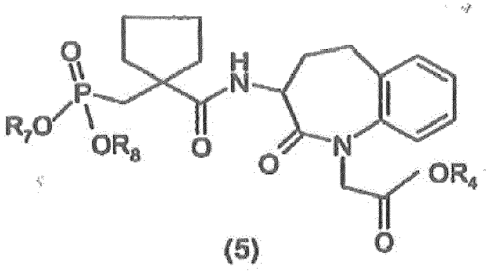 dans laquelle les symboles R₄ ,et R₇ à R₈ ont la signification donnée dans la revendication 1, tous leurs stéréoisomères et sels pharmaceutiquement acceptables.

4. Le composé à utilisation selon la revendication 1, **caractérisé en ce que** le composé à utilisation est acide (2R)-2-{[1-({[(3S)-1-(carboxyméthyl)-2-oxo-2,3,4,5-tétrahydro-1H-1-benzazépin-3-yl]amino}carbonyle)cyclopent-yl]méthyl}-4-phénylbutanoïque de formule (6), 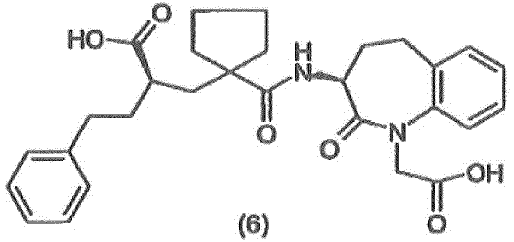 ainsi que ses sels pharmaceutiquement acceptables.

5. Le composé à utilisation selon la revendication 1, **caractérisé en ce que** le composé à utilisation est acide (2R)-2-{[1-({[(3S)-1-(carboxyméthyl)-2-oxo-2,3,4,5-tétrahydro-1H-1-benzazépin-3-yl]amino}carbonyle)cyclopent-yl]méthyl}-4-(1-naphthyl)butanoïque de formule (7), 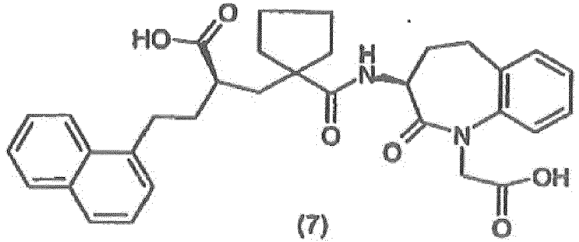 ainsi que ses sels pharmaceutiquement acceptables.

6. Le composé à utilisation selon la revendication 1, **caractérisé en ce que** le composé à utilisation est tert-Butyl-((3S)-3-{[(1-{[(benzyloxy)éthoxy)phosphoryl]méthyl}cyclopentyl)-carbonyl]amino)-2-oxo-2,3,4,5-tétrahydro-1*H*-1-benzazépin-1-yl)acétate de formule (8), 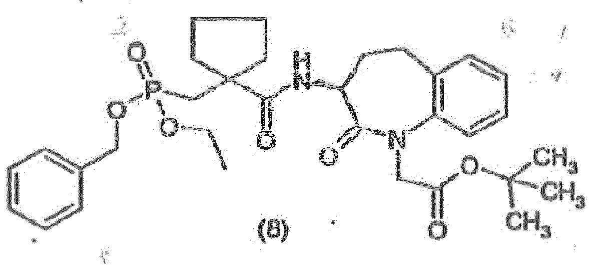 ainsi que ses sels pharmaceutiquement acceptables.

7. Le composé à utilisation selon l'une quelconque des revendications 1 à 6, **caractérisé en ce que** le sel pharmaceutiquement acceptable est choisi dans le groupe constitué du sel de lithium, du sel de calcium, du sel de magnésium et du sel de zinc, de préférence **en ce que** le sel pharmaceutiquement acceptable est le sel de calcium.

8. Le composé à utilisation selon l'une quelconque des revendications 1 à 7, **caractérisé en ce que** le composé est destiné à utilisation dans la prophylaxie et/ou le traitement de (i) toutes les formes de glaucome primaire et secondaire, de préférence le glaucome primaire à angle ouvert, glaucome à tension normale, glaucome primitif à angle fermé, syndrome de pseudoexfoliation et glaucome, syndrome de dispersion pigmentaire et glaucome, glaucome néovasculaire, glaucome inflammatoire, glaucome lié au cristallin, glaucome traumatique, glaucome congénital primitif, glaucome d'origine iatrogène ou glaucome malin.

9. Le composé à utilisation selon l'une quelconque des revendications 1 à 7, **caractérisé en ce que** le composé est destiné à utilisation dans la prophylaxie et/ou le traitement de (ii) maladies maculaires acquises, de préférence dégénérescence maculaire liée à l'âge, néovascularisation choroïdienne idiopathique, choriorétinopathie séreuse centrale, troubles de l'interface vitréomaculaire, télangiectasie maculaire idiopathique, oedème maculaire cystoïde ou oedème maculaire microkystique.

10. Le composé à utilisation selon l'une quelconque des revendications 1 à 7, **caractérisé en ce que** le composé est destiné à utilisation dans la prophylaxie et/ou le traitement de (iii) neuropathie optique, de préférence neuropathie optique ischémique antérieure ou postérieure; ou de (iv) névrite optique; ou de (v) uvéite, de préférence une uvéite antérieure, une uvéite intermédiaire, une uvéite postérieure ou une panuvéite; ou de (viii) sclérite et épisclérite.

11. Le composé à utilisation selon l'une quelconque des revendications 1 à 7, **caractérisé en ce que** le composé est destiné à utilisation dans la prophylaxie et/ou le traitement de (vi) dystrophies héréditaires du fond d'oeil, de préférence dystrophie de cône, dystrophie cône-bâton, dystrophie bâtonnet, maladie de Stargardt, dystrophie cornéo-rétinienne cristalline de Bietti, taches rétiniennes bénignes familiales, dystrophie maculaire vitelliforme de Best, dystrophie maculaire vitelliforme adulte, dystrophie maculaire de Caroline du Nord, drusen dominant familial ou dystrophie maculaire annulaire concentrique.

12. Le composé à utilisation selon l'une quelconque des revendications 1 à 7, **caractérisé en ce que** le composé est destiné à utilisation dans la prophylaxie et/ou le traitement de (vii) maladies vasculaires de la rétine, de préférence rétinopathie diabétique, rétinopathie non diabétique, maladie occlusive veineuse rétinienne, maladie occlusive artérielle rétinienne, syndrome d'ischémie oculaire, maladie oculaire hypertensive, rétinopathie falciforme, rétinopathie thalassémique, rétinopathie du prématuré, macroanévrisme de l'artère rétinienne, télangiectasie rétinienne primaire, maladie d'Eales ou rétinopathie radiologique.

13. Le composé à utilisation selon l'une quelconque des revendications 1 à 7, **caractérisé en ce que** le composé est destiné à utilisation dans la prophylaxie et/ou le traitement de (xii) myopie et myopie dégénérative; ou de (xiv) maladie de l'oeil sec.

14. Le composé à utilisation selon l'une quelconque des revendications 1 à 7, **caractérisé en ce que** le composé est destiné à utilisation dans la prophylaxie et/ou le traitement de (x) traumatisme du globe oculaire; ou de (xiii) traumatisme postopératoire, de préférence traumatisme mécanique dû à une chirurgie conventionnelle, traumatisme thermique dû à une chirurgie au laser ou traumatisme dû à la cryochirurgie; ou de (xi) opacités vitréennes, de préférence hémorragie vitréenne ou hyalose d'astéroïde; ou de (ix) décollements de rétine.

15. Le composé à utilisation selon l'une quelconque des revendications 1 à 7, **caractérisé en ce que** le composé est destiné à utilisation dans la prophylaxie et/ou le traitement de (xv) maladies cornéennes, de préférence abrasions, lacérations, ulcères, dystrophies, opacités, décompensation endothéliale et épithéliale, oedème postopératoire, dégénérescence cornéenne ou vascularisation cornéenne; ou d'une ectasie cornéenne, de préférence un kératocône.
